# EUROPÄISCHE PATENTANMELDUNG

(11) **EP 0 734 714 A2**
(43) Veröffentlichungstag der Anmeldung: **02.10.1996**
(21) Anmeldenummer: 96104378.3
(22) Anmeldetag: 20.03.1996
(51) Int. Cl.: A61K 7/06, A61K 7/11

(54) **Verwendung von carboxylgruppenhaltigen Polykondensaten als Hilfsmittel in kosmetischen Zubereitungen**

(30) Priorität: 27.03.1995 DE 19510684
(71) Anmelder: BASF Aktiengesellschaft, 67063 Ludwigshafen (DE)
(72) Erfinder: Kim, Son Nguyen, Dr., 69502 Hemsbach (DE); Sanner, Axel, Dr., 67227 Frankenthal (DE); Hössel, Peter, Dr., 67105 Schifferstadt (DE)

(57) **Zusammenfassung**

Verwendung von carboxylgruppenhaltigen wasserdispergierbaren Polykondensationsprodukten mit Glasübergangstemperaturen Tg > 20°C und Säurezahlen von 30 bis 160, erhältlich durch Kondensation von
A) 0,3 bis 15 mol-% mindestens einer Verbindung ausgewählt aus der Gruppe aus 2,2-Dimethylolpropansäure, 1,3,5-Benzoltricarbonsäure und deren C₁-C₈-Alkylestern und Säurechloriden und 5-Hydroxyisophthalsäuren und deren Acylderivaten, und
B) 85 bis 99,7 mol-% einer Mischung aus
   B1) einem gesättigten Diol oder einem C₂-C₈-Diamin oder deren Mischungen, und
   B2) mindestens einer Verbindung aus der Gruppe der Monohydroxycarbonsäuren und deren Lactonen, der Dicarbonsäuren ausgenommen der unter A) genannten Isophthalsäurederivate, und der Aminocarbonsäuren,
als Hilfsmittel in kosmetischen Zubereitungen.

## Beschreibung

Die vorliegende Erfindung betrifft die Verwendung von carboxylgruppenhaltigen wasserdispergierbaren Polykondensationsprodukten mit Glasübergangstemperaturen Tg > 20°C und Säurezahlen von 30 bis 160, erhältlich durch Kondensation von
A) 0,3 bis 15 mol-% mindestens einer Verbindung ausgewählt aus der Gruppe aus 2,2-Dimethylolpropansäure, 1,3,5-Benzoltricarbonsäure und deren C₁-C₈-Alkylestern und Säurechloriden, 5-Hydroxyisophthalsäuren und deren 5-Acylderivaten, und
B) 85 bis 99,7 mol-% einer Mischung aus
   B1) einem gesättigten Diol oder einem C₂-C₈-Diamin oder deren Mischungen, und
   B2) mindestens einer Verbindung aus der Gruppe der Monohydroxycarbonsäuren und deren Lactonen, der Dicarbonsäuren ausgenommen der unter A) genannten Isophthalsäurederivate, und der Aminocarbonsäuren,
als Hilfsmittel in kosmetischen Zubereitungen, insbesondere in Haarbehandlungsmitteln. Weiterhin betrifft die Erfindung Haarbehandlungsmittel, enthaltend Polykondensationsprodukte als Filmbildner.

Aufgrund der ständig wachsenden Forderung nach umweltverträglicheren Einsatzstoffen soll der Gehalt an flüchtigen organischen Bestandteilen (volatile organic compounds - VOC) auch in kosmetischen Zubereitungen wie beispielsweise Haarspray möglichst niedrig gehalten werden bzw. gesenkt werden. Das bedeutet, daß der Alkohol als Lösungsmittel zunehmend durch Wasser ersetzt werden soll.

Bei Verwendung herkömmlicher Haarfestigungspolymere kann dies jedoch Schwierigkeiten bereiten, da bei höheren Wassergehalten Viskosität und Sprühverhalten der Haarfestigungsmittel und die Trocknung der Polymerfilme nicht befriedigend sind.

Aus der US-A 4 300 580 sind Natriumsulfonat-gruppenhaltige lineare Polykondensate bekannt, die sich zur Verwendung in Haarbehandlungsmitteln eignen. Solche Polykondensate weisen jedoch eine schlechte Verträglichkeit mit organischen Lösungsmitteln und lassen hinsichtlich der Auswaschbarkeit zu wünschen übrig.

Weiterhin ist aus der DE-AS 26 33 418 und der DE-AS 25 06 461 bekannt, Natriumsulfonat-haltige verzweigte Polyester in Haarbehandlungsmitteln einzusetzen. Auch diese Mittel weisen keine befriedigende Verträglichkeit mit organischen Lösungsmitteln auf.

In der DE-OS 42 24 761 werden carboxylgruppenhaltige verzweigte Polykondensate für den Einsatz in kosmetischen Zubereitungen beschrieben, die auf Basis von aromatischen Carbonsäuren mit vicinalen Carbonsäuregruppen erhalten werden. Diese Polykondensate weisen hinsichtlich der Hydrolysebeständigkeit einige Nachteile auf.

Aufgabe der vorliegenden Erfindung war es, Polymere für den Einsatz als Filmbildner in Haarfestigungsmitteln zu finden, die erhöhte Wasseranteile in den entsprechenden kosmetischen Zubereitungen ermöglichen, ohne daß eine Viskositätserhöhung bewirkt oder andere anwendungstechnische Eigenschaften wie das Sprühverhalten negativ beeinflußt werden. Nach der Applikation auf das Haar sollen außerdem klebfreie, klare Filme mit guter Festigungswirkung und guter Auswaschbarkeit erhalten werden.

Demgemäß wurde die Verwendung der eingangs definierten Polykondensationsprodukte sowie diese enthaltende kosmetische Zubereitungen gefunden.

Die Polykondensationsprodukte sind durch Kondensation der folgenden Komponenten erhältlich.

Als Komponenten (A) werden trifunktionelle Verbindungen, ausgewählt aus der Gruppe bestehend aus 2,2-Dimethylolpropansäure, 1,3,5-Benzol-tricarbonsäure, 1,3,5-Benzol-tricarbonsäure-mono- oder trialkylestern mit C₁-C₈-Alkylresten, Säurechloriden der 1,3,5-Benzoltricarbonsäure und 5-Acyloxyisophthalsäuren, bevorzugt 5-Acetoxyisophthalsäure eingesetzt. Besonders bevorzugte Komponenten A) sind 2,2-Dimethylolpropansäure und 1,3,5-Benzoltricarbonsäure. Verwendet man Ester oder Säurechloride der Benzoldicarbonsäure, so werden diese unter den Bedingungen der Kondensation umgeestert, so daß sie auch trifunktionelle Verbindungen darstellen.

Die Komponenten A) werden in Mengen von 0,3 bis 15 mol-%, bevorzugt 1 bis 12 mol-%, bezogen auf die Gesamtmenge an Ausgangsverbindungen, eingesetzt.

Als Komponenten B) werden 85 bis 99,7, vorzugsweise 88 bis 99 mol-% einer Mischung aus den B1) und B2) eingesetzt, wobei B1) vorzugsweise in Mengen von 10 bis 49 mol-%, B2) in Mengen von 30 bis 80 mol-%, jeweils bezogen auf die Gesamtmenge an A) und B), eingesetzt. Das Verhältnis von eingesetzten Säuregruppen zu eingesetzten Hydroxylgruppen soll vorzugsweise 0,95 : 1 bis 1,2 : 1 betragen.

Als Komponenten B1) kommen eine oder mehrere Verbindungen, ausgewählt aus der Gruppe der gesättigten Diole und der gesättigten diprimären C₂-C₈-Diamine in Betracht. Geeignete Diole sind beispielsweise C₂-C₈-Diole wie Ethylenglykol, Diethylenglykol, 1,2- und 1,3-Propandiol, 1,2-Butandiol, 1,4-Butandiol, Neopentylglykol, 1,4-Cyclohexyldimethylol oder Polyetherdiole mit Molekulargewichten bis 500 wie Polyoxyethylenglykol, Polyoxypropylenglykol oder Polytetrahydrofuran. Geeignete Diamine sind beispielsweise Ethylendiamin, 1,4-Butylendiamin oder 1,6-Hexamethylendiamin.

Als Komponenten B2) kommen Verbindungen, ausgewählt aus der Gruppe der Hydroxycarbonsäuren, Dicarbonsäuren und Aminocarbonsäuren in Betracht. Geeignete Monohydroxycarbonsäuren sind beispielsweise Milchsäure, 2-Hydroxyessigsäure oder 3-Hydroxypivalinsäure oder Lactone wie ε-Caprolacton. Geeignete Dicarbonsäuren, die von der unter A) genannten 5-Hydroxyisophthalsäure und deren Derivate verschieden sind, sind beispielsweise einkernige aromatische Dicarbonsäuren, beispielsweise Phthalsäure, Trimellithsäure, Terephthalsäure oder Isophthalsäure, die bevorzugt jedoch keine vicinalen Carbonsäuregruppen tragen, wobei Isophthalsäure besonders bevorzugt ist. Weiterhin eignen sich Cycloalkan- oder Alkandicarbonsäuren mit 2 bis 8 C-Atomen, beispielsweise Adipinsäure, Bernsteinsäure oder 1,4-Cyclohexyldicarbonsäure. Als Komponenten B2) kommen auch Aminocarbonsäuren mit 2 bis 8 C-Atomen oder deren Lactame, beispielsweise ε-Caprolactam in Betracht.

Zusätzlich können noch bis zu 3 Mol-%, bezogen auf die Gesamtmolzahl von Ausgangsverbindungen, einer weiteren trifunktionellen Verbindung oder bis zu 1 mol-% eines Diisocyanats zur Molekulargewichtserhöhung zugegeben werden. Geeignete trifunktionelle Verbindungen sind gesättigte Triole wie Glycerin oder Trimethylolpropan. Geeignete Diisocyanate sind beispielsweise Isophorondiisocyanat oder Hexamethylendiisocyanat.

Die molaren Anteile der Ausgangskomponenten werden bevorzugt so bemessen, daß die resultierenden Polykondensationsprodukte Säurezahlen von 30 bis 160 mg KOH/g Substanz, vorzugsweise 40 bis 100, aufweisen.

Bevorzugte Polykondensationsprodukte sind erfindungsgemäß Polyester.

Die Herstellung der Polykondensationsprodukte kann auf an sich bekannte Weise erfolgen. Üblicherweise kann die Kondensation als Schmelzkondensation bei Temperaturen im Bereich von 160 bis 260°C während eines Zeitraums von mehreren Stunden (beispielsweise 5 bis 20 h) unter einer Stickstoffatmosphäre bei Drücken von 0,2 bis 2 bar, bevorzugt unter Normaldruck, durchgeführt werden, wobei das entstehende Reaktionswasser abdestilliert wird. Gegen Ende der Reaktion kann noch im Produkt vorhandenes Reaktionswasser durch Destillation bei 1 bis 100 mbar bei Temperaturen im Bereich von 210 bis 230°C entfernt werden. Es kann sich auch empfehlen, geringe Mengen an üblichen Katalysatoren zu dem Reaktionsgemisch zu geben, beispielsweise Tetraisopropylorthosilicat, wobei üblicherweise Mengen im Bereich von 10 bis 100 ppm gewählt werden.

Die so erhaltenen wasserdispergierbaren oder wasserlöslichen Polykondensationsprodukte sind farblose bis honiggelbe Produkte mit relativ geringer Viskosität und K-Werten (gemessen 1 %ig in N-Methylpyrrolidon) nach Fikentscher, im Bereich vom 20 bis 40. Die Glasübergangstemperaturen betragen > 20°C.

Die Polykondensationsprodukte werden in wäßrigen Dispersionen vorzugsweise in partiell oder vollständig neutralisierter Form, mit beispielsweise 2-Amino-2-methylpropanol als Neutralisierungsmittel, verwendet.

Die sulfonatgruppenfreien Polykondensationsprodukte werden erfindungsgemäß als Hilfsmittel in kosmetischen Zubereitungen wie Hautcremes, Hautlotionen oder Nagellack und insbesondere in haarkosmetischen Zubereitungen verwendet, vor allem in Haarfestigungsmitteln wie Haarsprays, Festigerlotionen oder Schaumfestigern.

Wegen der guten Wasserverträglichkeit lassen sich rein wäßrige Zubereitungen herstellen, wobei die gute Hydrolysebeständigkeit von Vorteil ist. Die Polykondensationsprodukte sind aber auch gut verträglich mit organischen Lösungsmitteln und organischen Treibmitteln.

Aufgrund der niedrigen Viskositäten können haarkosmetische Zubereitungen mit hohen Feststoffgehalten hergestellt werden, so daß weniger Lösungsmittel benötigt wird.

Neben der guten Umweltverträglichkeit der Produkte, bei denen es keine Restmonomerenproblematik gibt, weisen sie aber auch gute filmbildende Eigenschaften auf.

In den erfindungsgemäßen haarkosmetischen Zubereitungen können die Polykondensationsprodukte sowohl als alleinige Filmbildner als auch als Mischungen mit herkömmlichen, für solche Zwecke geeigneten Polymeren eingesetzt werden, wobei sich Mischungsverhältnisse von Polykondensaten zu herkömmlichen Polymeren von 1 : 0,1 bis 1 : 2 empfehlen. Als herkömmliche Haarfestigungspolymere eignen sich beispielsweise anionische Polymere wie Acrylsäure- oder Methacrylsäure-Homopolymere oder -Copolymere, Copolymerisate aus Acrylsäure und Acrylamiden und Copolymere auf der Basis von Alkylvinylethern und Maleinsäuremonoalkylestern, amphotere Polymere wie Copolymerisate aus Octylacrylamid, Acrylat und Butylaminoethylmethacrylat sowie nichtionische Polymere wie Vinylpyrrolidon-Homopolymere, Vinylpyrrolidon-Vinylacetat-Copolymere und Copolymerisate aus Vinylpyrrolidon, Vinylacetat und Vinylpropionat verwendet.

Insbesondere eignen sich Kombinationen der Polykondensationsprodukte mit Polyvinylcaprolactam.

Die erfindungsgemäßen haarkosmetischen Zubereitungen können die Polykondensationsprodukte in Mengen von 0,1 bis 25, bevorzugt 1 bis 15 Gew.-% enthalten.

Die erfindungsgemäßen Zubereitungen können als rein wäßrige Zubereitungen angewendet werden oder in wäßrig-alkoholischer Lösung mit Ethanol oder Isopropanol mit oder ohne Treibmittel. Als Treibmittel eignet sich beispielsweise Dimethylether. Die erfindungsgemäßen Zubereitungen werden vorzugsweise mit bis zu 80 Gew.-% an flüchtigen organischen Lösungsmitteln formuliert.

Weiterhin können die haarkosmetischen Zubereitungen übliche Hilfsstoffe wie beispielsweise Tenside, Emulgatoren oder Duftstoffe enthalten.

### Beispiele 1 bis 10

### Allgemeine Vorschrift zur Herstellung der Polykondensate

Eine Mischung der Ausgangsverbindungen (Mengen s. Tabelle) und 50 ppm Tetraisopropylorthosilicat wurde unter einer Stickstoffatmosphäre unter Rühren auf Temperaturen von 160 bis 180°C aufgeheizt und 3 h bei dieser Temperatur gehalten, wobei das entstehende Reaktionswasser abdestilliert wurde. Die klare Schmelze wurde noch 15 h auf 200°C erhitzt, dann wurde die Temperatur stündlich um 15°C gesteigert, bis sie im Bereich von 220-240°C lag. Anschließend wurde noch 2 h bei 20 mbar erhitzt, wobei Reste des Reaktionswassers entfernt wurde. Nach dem Abkühlen erhielt man klare hellgelbe bis gelbe Produkte.

**Tabelle**

| | Zusammensetzung [mol] | | | | | | | | | | | |
|---|---|---|---|---|---|---|---|---|---|---|---|---|
| Bsp. Nr. | BTS | DMP A | IPS | ADS | NPG | CHD M | DEG | PPG 450 | Mis | ε-CL. | Tg [°C] | SZ |
| 1 | 1 | - | - | - | 1,2 | - | - | - | 6 | - | 64 | 89,7 |
| 2 | 1,5 | - | 8, 5 | - | 5 | - | 5 | - | - | - | 59 | 69,8 |
| 3 | 1,2 5 | - | 8, 7 5 | - | 5 | - | 5 | - | 5 | - | 56 | 84,5 |
| 4 | 1 | - | 8, 6 | - | 5 | - | 5 | - | - | 2 | 48 | 69 |
| 5 | 0,6 | 0,4 | 8, 4 | 0,2 | 5 | - | 5 | - | 3 | 1 | 53 | 85 |
| 6 | - | 2,5 | 11 | - | 8,5 | - | 1 | - | 10 | - | 57 | 94 |
| 7 | - | 1,5 | 10 | - | 8 | - | 1,5 | - | 3 | - | 59 | 78 |
| 8 | - | 1 | 9, 6 | - | 7 | 1 | 0,5 | - | - | - | 59 | 65,5 |
| 9 | - | 1 | 10 | 0,5 | 7,5 | 1 | 0,5 | - | - | - | 54 | 69 |
| 10 | - | 1,5 | 10 | 0,8 | 9 | 0,2 | - | 0,5 | - | - | 53 | 72 |
| BTS: 1,3,5-Benzoltricarbonsäure DMPA: 2,2-Dimethylolpropansäure IPS: Isophthalsäure ADS: Adipinsäure NPG: Neopentylglykol CHDM: 1,4-Cyclohexyldimethylol DEG: Diethylenglykol PPG 450: Polypropylenglykol M_{w}=450 Mis: Milchsäure ε-CL: ε-Caprolacton Tg: Glastemperatur wurde durch Differentialthermoanalyse Methode ASTM D 3418 ermittelt. SZ: Säurezahl, [mg KOH/g Substanz] | | | | | | | | | | | | |

### Formulierungsbeispiele:

| 1) Aerosol-Haarspray: (VOC⁺⁾80) | |
|---|---|
| Polymer gemäß Beispiel 5 | 3,00 Gew.-% |
| 2-Amino-2-methylpropanol | 0,43 Gew.-% |
| Wasser dest. | 16,67 Gew.-% |
| Ethanol | 45,00 Gew.-% |
| Dimethylether | 35,00 Gew.-% |
| Parfümöl, Tensid | q.s.⁺⁺⁾ |

| | |
|---|---|
| ⁺⁾ VOC = Volatile Organic Content | |
| q.s⁺⁺⁾ nach Belieben | |

entsprechende VOC 80-Formulierungen können mit Polymer 6 bis 10 enthalten werden.

| 2) Handpumpen-Haarspray (VOC 55) | |
|---|---|
| Polymer gemäß Beispiel 5 | 4,00 Gew.-% |
| 2-Amino-2-methylpropanol | 0,57 Gew.-% |
| Wasser dest. | 40,43 Gew.-% |
| Ethanol | 55,00 Gew.-% |
| Parfümöl, Tensid | q.s.⁺⁺⁾ |

| | |
|---|---|
| q.s⁺⁺⁾ nach Belieben | |

entsprechende VOC 55-Formulierungen können mit Polymer 6 bis 10 enthalten werden.

| 3) Alkohol-frei Handpumpen-Haarspray (VOC 0) | |
|---|---|
| Polymer gemäß Beispiel 10 (neutralisiert mit Aminomethylpropanol) | 4,00 Gew.-% |
| Polyvinylcaprolactam (K-Wert 35/1%ige DMF) | 4,00 Gew.-% |
| Wasser dest. | 82,00 Gew.-% |
| Parfümöl, Tensid | q.s.⁺⁺ |

| | |
|---|---|
| q.s⁺⁺⁾ nach Belieben | |

entsprechende VOC 0-Formulierungen können mit Polymer 6 bis 9 enthalten werden.

## Patentansprüche

1. Verwendung von sulfonatgruppenfreien carboxylgruppenhaltigen wasserdispergierbaren Polykondensationsprodukten mit Glasübergangstemperaturen Tg > 20°C und Säurezahlen von 30 bis 160, erhältlich durch Kondensation von
A) 0,3 bis 15 mol-% mindestens einer Verbindung ausgewählt aus der Gruppe aus 2,2-Dimethylolpropansäure, 1,3,5-Benzoltricarbonsäure und deren C₁-C₈-Alkylestern und Säurechloriden, und 5-Hydroxyisophthalsäuren und deren Acylderivaten, und
B) 85 bis 99,7 mol-% einer Mischung aus
B1) einem gesättigten Diol oder einem C₂-C₈-Diamin oder deren Mischungen, und
B2) mindestens einer Verbindung aus der Gruppe der Monohydroxycarbonsäuren und deren Lactonen, der Dicarbonsäuren ausgenommen der unter A) genannten Isophthalsäurederivate, und der Aminocarbonsäuren,
als Hilfsmittel in kosmetischen Zubereitungen.

2. Verwendung nach Anspruch 1, wobei für die Polykondensationsprodukte 1 bis 12 mol-% der Komponente A) eingesetzt werden.

3. Verwendung nach Anspruch 1 oder 2, wobei als Polykondensationsprodukte Polyester verwendet werden.

4. Verwendung nach einem der Ansprüche 1 bis 3 in haarkosmetischen Zubereitungen.

5. Verwendung nach Anspruch 4 als Haarfestigerpolymere.

6. Haarkosmetische Zubereitungen, enthaltend als Filmbildner carboxylgruppenhaltige Polykondensationsprodukte mit Glasübergangstemperaturen Tg > 20°C und Säurezahlen von 30 bis 160, erhältlich durch Kondensation von
A) 0,3 bis 15 mol-% mindestens einer Verbindung ausgewählt aus der Gruppe aus 2,2-Dimethylolpropansäure, 1,3,5-Benzoltricarbonsäure und deren C₁-C₈-Alkylestern und Säurechloriden und 5-Hydroxyisophthalsäuren, und
B) 85 bis 99,7 mol-% einer Mischung aus
B1) einem gesättigten Diol oder einem C₂-C₈-Diamin oder deren Mischungen, und
B2) mindestens einer Verbindung aus der Gruppe der Monohydroxycarbonsäuren und deren Lactonen, der Dicarbonsäuren ausgenommen 5-Hydroxyisophthalsäuren, und der Aminocarbonsäuren.

7. Zubereitungen nach Anspruch 6, enthaltend die Polykondensationsprodukte in Mengen von 0,1 bis 25 Gew.-%, bezogen auf den Feststoffgehalt.

8. Zubereitungen nach Anspruch 6, enthaltend die Polykondensationsprodukte in Form wäßriger Dispersionen oder wäßrig-ethanolischer Lösungen sowie übliche haarkosmetische Hilfsmittel.

9. Zubereitungen nach Anspruch 6 oder 7, enthaltend als Filmbildner Polyester.

10. Zubereitungen nach einem der Ansprüche 6 bis 9, enthaltend zusätzlich weitere für die Haarkosmetik übliche Polymere.
